# EUROPEAN PATENT APPLICATION

(11) **EP 4 759 257 A1**
(43) Date of publication of application: **17.06.2026**
(21) Application number: 25223194.9
(22) Date of filing: 13.12.2025
(51) Int. Cl.: A61B 18/12, A61B 18/14, A61B 1/00

(54) **FLEXIBLE ELECTRODE PULSED ABLATION CATHETER, PULSED ABLATION DEVICE, AND ABLATION EQUIPMENT**

(30) Priority: 13.12.2024 CN 202411845219
(71) Applicant: Merryspring Medical Technology (Zhejiang) Co, Ltd., Jiaxing, Zhejiang (CN)
(72) Inventor: FU, Jiawei, Jiaxing (CN); TAO, Lin, Jiaxing (CN); TANG, Zhongchao, Jiaxing (CN); XU, Shuhan, Jiaxing (CN)
(74) Representative: De Arpe Tejero, Manuel

(57) **Abstract**

Provided are a flexible electrode pulsed ablation catheter, a pulsed ablation device, and an ablation equipment. The ablation catheter comprises: a tube body with a distal end and a proximal end, the tube body comprising a traction structure and a delivery tube assembly sleeved on the traction structure; an electrode assembly, the electrode assembly comprising at least one connecting tube and at least two electrodes, with adjacent electrodes fixedly connected by the connecting tube, the electrodes being tubular structures with internal cavities; one end of the traction structure extends through the delivery tube assembly into the electrode assembly and is fixedly connected to one end of the electrode near the distal end of the tube body; under external force, the traction structure can move the relative ends of the same electrode towards or away from each other, causing the tubular structure to contract or expand.

## Description

### TECHNICAL FIELD

This application relates to the field of ablation technology, specifically a flexible electrode pulsed ablation catheter, a pulsed ablation device, and an ablation equipment.

### BACKGROUND ART

The pulsed ablation device operates on the principle of pulsed ablation electric fields, which involves applying short, high-voltage pulses between two electrodes crafted from special materials. This process alters the native membrane potential of cells and induces irreversible nanoscale pores within the lipid bilayer of their membranes, thereby disrupting cellular homeostasis and ultimately leading to cell death. Notably, this ablation technique selectively targets cells within a specific region, while preserving the integrity of the cellular tissue scaffold and fibrous structures. Importantly, tissues adjacent to the ablation zone, such as blood vessels or surrounding healthy tissues, remain unaffected, thereby mitigating the "heat sink effect" commonly associated with ablation areas and facilitating tissue repair within the organism.

Pulsed ablation devices are mainly configured for atrial fibrillation treatment, tumor treatment, and cardiac electrophysiological disease treatment, comprising liver cancer, lung cancer, kidney cancer, etc. Existing pulsed ablation devices have several shortcomings in lung tumor treatment, limiting their clinical application: for example, at least two and up to six electrodes need to be placed inside or around the tumor, which is time-consuming and may lead to incorrect needle placement, with a high risk of complications such as pneumothorax and pleural effusion due to multiple punctures; the ablation electrodes must be placed precisely and parallel to generate a sufficiently strong electric field. On one hand, the angle of the electrodes may cause overcurrent at the electrode proximity, leading to unnecessary Joule heating; on the other hand, incomplete ablation may occur at positions where the electrodes are far apart. In the chest, parallel placement of electrodes is extremely challenging due to the obstruction of ribs; the air in the lung alveoli is an excellent insulator for current, significantly affecting energy transfer between electrodes, limiting the ablation extent of external lung cancer tissue, leading to overestimation of the ablation area, and reducing ablation effectiveness.

### SUMMARY

To solve the above technical problems, this application improves the flexibility of the electrode assembly, achieving greater contact area between the electrode and the lesion, enabling delivery and ablation of lesions at large peripheral angles, ensuring ablation effectiveness, and increasing ablation success rate.

This application provides a flexible electrode pulsed ablation catheter, comprising:
a tube body with a distal end and a proximal end, the tube body comprising a traction structure and a delivery tube assembly sleeved on the traction structure;
an electrode assembly located at the distal end of the tube body, the electrode assembly comprising at least one connecting tube and at least two electrodes, with adjacent electrodes fixedly connected by the connecting tube, the electrodes being tubular structures with internal cavities;
wherein one end of the traction structure extends through the delivery tube assembly into the electrode assembly and is fixedly connected to one end of the electrode near the distal end of the tube body;
wherein the traction structure moves axially along the delivery tube assembly under external force to bring the relative ends of the same electrode towards or away from each other, causing the tubular structure to contract or expand.

Further, the electrode comprises at least one of the following features:
the electrode in a contracted state has a maximum diameter of 0.5mm-5mm;
the electrode in an expanded state has a maximum diameter of 1.5mm-20mm.

Further, the electrode in a contracted state has a cylindrical cross-section; the electrode in an expanded state has an elliptical cross-section.

Further, the electrode assembly comprises at least one of the following features:
the spacing between adjacent electrodes is 5mm-20mm;
the length of the electrode assembly is 10mm-40mm.

Further, adjacent electrodes have opposite polarities.

Further, the tubular structure has a first aggregation end and a second aggregation end;
wherein the first aggregation end and the second aggregation end are arranged along the axial direction of the delivery tube assembly, the first aggregation end is provided near the distal end of the tube body, and the second aggregation end is provided near the proximal end of the tube body;
wherein one end of the delivery tube assembly is fixedly connected to the second aggregation end of the electrode near the proximal end of the tube body;
wherein one end of the traction structure extends through the delivery tube assembly into the electrode assembly and is fixedly connected to the first aggregation end of the electrode near the distal end of the tube body.

Further, the electrode is a tubular structure woven from metal wires.

Further, the electrode further comprises an extended segment, the extended segment is fixedly connected at both ends to the first aggregation end and the second aggregation end;
wherein the extended segment comprises at least two first connecting members, the at least two first connecting members are spaced around the circumference of the delivery tube assembly.

Further, when the electrode is in a compressed state, the length direction of the first connecting member is parallel to the axial direction of the delivery tube assembly, the thickness direction of the first connecting member passes through the axis of the delivery tube assembly, and the first connecting member comprises at least one of the following features:
the thickness of the first connecting member is much smaller than its width;
the width of the first connecting member is much smaller than its length; and
the number of first connecting members is 2-14.

Further, the surface slope of the first connecting member gradually increases from small to large and then gradually decreases from large to small, forming an arched structure along the first converged end to the second converged end.

Further, it further comprises an extended segment, with both ends of the extended segment fixedly connected to the first converged end and the second converged end, respectively;
wherein the extended segment comprises a balloon membrane and an electrode membrane disposed sequentially from the inside out. The interior of the balloon membrane has a sealed cavity that can communicate with the delivery tube assembly, allowing liquid to enter or exit the sealed cavity through the delivery tube assembly, thereby driving the balloon membrane to contract or expand.

Further, the electrode membrane comprises at least two second connecting members disposed at intervals around the circumference of the balloon membrane.

Further, the electrode membrane comprises at least one of the following features:
the thickness of the electrode membrane is 10µm-100µm;
the electrode membrane is a multilayer membrane structure.

Further, when the electrode is in a compressed state, the length direction of the second connecting member is parallel to the axial direction of the delivery tube assembly, and the thickness direction of the second connecting member passes through the axis of the delivery tube assembly. The second connecting member comprises at least one of the following features:
the thickness of the second connecting member is much smaller than its width;
the width of the second connecting member is much smaller than its length; and
the number of second connecting members is 2-10.

Further, the electrode assembly also comprises a cap and a rigid electrode. The first end of the cap is provided with an arcuate surface, and the second end of the cap is provided with a traction structure mounting position and a converged end mounting position.

The traction structure mounting position is configured for fixedly connecting with one end of the traction structure, and the converged end mounting position is configured for fixedly connecting with the first converged end of an electrode disposed near the distal end of the tube body.

The first end of the rigid electrode is fixedly connected to the second converged end of an electrode disposed near the proximal end of the tube body, and the second end of the rigid electrode is fixedly connected to the tube body.

Further, the cap comprises an end cap and an electrode ring. The arcuate surface is disposed at the first end of the end cap, and the electrode ring is fixedly connected to the second end of the end cap. After the electrode ring is connected to the second end of the end cap, the converged end mounting position is formed between the electrode ring and the end cap. The traction structure mounting position is disposed at the second end of the end cap.

Further, the stiffness of the tube body gradually decreases from the proximal end to the distal end of the tube body.

Moreover, the tube body is made of a radiopaque material.

Further, , the delivery tube assembly sequentially comprises an insulating tube, a channel tube, an outer catheter, and a stress tube along the radial direction of the delivery tube assembly.

The first end of the outer catheter is fixedly connected to the electrode assembly, and the second end of the outer catheter can be fixedly connected to a handle assembly. The stress tube is disposed at the connection position between the outer catheter and the handle assembly.

Part of the wires electrically connected to the electrodes can extend into the insulating tube, and the insulating tube is configured to isolate the wires electrically connected to adjacent electrodes from each other.

A gap is provided between the inner wall of the channel tube and the outer wall of the insulating tube, and one end of the channel tube communicates with the electrode assembly to direct liquid to the location of the electrode assembly through the channel tube.

Further, it also comprises at least one position sensing device. The at least one position sensing device is disposed inside the electrode and fixedly mounted on the traction structure.

The second aspect of this application also protects a pulsed ablation device, comprising a flexible electrode pulsed ablation catheter with a handle assembly as described above.

The first end of the handle assembly can be electrically connected to an ablation energy-emitting ablation mechanism.

One end of the delivery tube assembly is fixedly connected to the second end of the handle assembly, and one end of the traction structure passes through the delivery tube assembly to be connected to the handle assembly in a driving manner. The handle assembly can drive the traction structure to reciprocate axially relative to the delivery tube assembly.

Further, the handle assembly comprises at least two wires, a rotation mechanism, and a connector assembly.

The rotation mechanism is fixedly connected to one end of the traction structure. Under external force, the rotation mechanism drives the traction structure to reciprocate axially relative to the delivery tube assembly.

The first ends of the at least two wires are electrically connected to the connector assembly, which is located at the first end of the handle assembly and electrically connected to the ablation mechanism.

The second ends of the at least two wires are respectively electrically connected to the at least two electrodes, to enable adjacent electrodes to have opposite polarities.

The third aspect of this application also protects a pulsed ablation apparatus, comprising an ablation mechanism and the pulsed ablation device described above. The ablation mechanism is configured to provide at least one pulse group to the pulsed ablation device.

Further, the pulse group comprises at least one high-frequency and high-voltage sub-pulse and/or one low-frequency and low-voltage sub-pulse. The high-frequency and high-voltage sub-pulse is a bipolar pulse, and the low-frequency and low-voltage sub-pulse is a unipolar pulse or a bipolar pulse. Among them, the bipolar pulse comprises positive pulse amplitude and negative pulse amplitude.

Further, the pulse group comprises at least one of the following features:
the voltage amplitude range of the bipolar pulses of the high-frequency and high-voltage sub-pulses is 2000V-70000V;
the pulse width of the bipolar pulses of the high-frequency and high-voltage sub-pulses is 50ns-50000ns;
the number of bipolar pulses of the high-frequency and high-voltage sub-pulses is 1-5000;
the amplitude voltage of the unipolar pulse and the bipolar pulse of the low-frequency and low-voltage sub-pulse is 200V-3000V;
the pulse width of the unipolar pulse and the bipolar pulse of the low-frequency and low-voltage sub-pulse is 10µs-500µs;
the number of unipolar pulses and bipolar pulses of the low-frequency and low-voltage sub-pulse is 1-5000; and
the number of pulse groups is 1-5000.

Implementing the embodiments of this application yields the following beneficial effects.

The electrode assembly, traction structure, and delivery tube assembly in this application cooperate to achieve synchronous contraction or expansion of at least two electrodes in the electrode assembly, ensuring that the flexible electrode pulsed ablation catheter can bend and expand along with the bronchial lumen, flexibly adjusting the electrode morphology to adapt to the lesion structure and achieving greater electrode contact area with the lesion. Moreover, the electrode assembly comprises at least two electrodes disposed at intervals. Compared to conventional non-extensible ring electrodes or needle electrode structures, this can reduce electrode stiffness, improve electrode extensibility, increase electrode coverage area, and enhance the flexibility of the electrode assembly, ensuring that the electrode assembly can enter lesions with larger bending angles. Additionally, adjacent electrodes are fixedly connected by connecting tubes to achieve synchronous contraction or expansion of adjacent electrodes, which can further enhance the flexibility of the electrode assembly, achieve greater electrode contact area with the lesion, facilitate delivery and ablation of lesions at large peripheral angles, ensure ablation efficacy, and improve ablation success rates.

### BRIEF DESCRIPTION OF THE DRAWINGS

To more clearly illustrate the technical solution of this application, the following provides a brief introduction to the drawings required for describing the embodiments or the prior art. It is evident that the drawings described below are merely some embodiments of this application. For those skilled in the art, other drawings can be obtained based on these drawings without exercising creative efforts.
Figure 1 is a structural diagram of a flexible electrode pulse ablation catheter in this embodiment;
Figure 2 is a structural diagram of the electrode assembly in a contracted state according to the first embodiment;
Figure 3 is a structural diagram of the electrode assembly in an expanded state according to the first embodiment;
Figure 4 is a structural diagram of the electrode in this embodiment;
Figure 5 is a cross-sectional view of the cap connected with the electrode in this embodiment;
Figure 6 is a structural diagram of the end cap in this embodiment;
Figure 7 is a structural diagram of the end cap, the electrode ring, and the electrode connected together in this embodiment;
Figure 8 is a structural diagram of the position sensing device connected with the electrode assembly in this embodiment;
Figure 9 is a structural diagram of the electrode assembly in a contracted state according to the second embodiment;
Figure 10 is a structural diagram of the electrode assembly in an expanded state according to the second embodiment;
Figure 11 is a structural diagram of the electrode assembly in a contracted state according to the third embodiment;
Figure 12 is a structural diagram of the electrode assembly in an expanded state according to the third embodiment;
Figure 13 is a cross-sectional view of the tube body in this embodiment;
Figure 14 is a structural diagram of the pulse ablation device in this embodiment;
Figure 15 is a schematic diagram of a pulse electric field energy packet that can be provided by a pulse ablation device in an embodiment;
Figure 16 is a schematic diagram of a pulse electric field energy packet that can be provided by a pulse ablation device according to the first embodiment;
Figure 17 is a schematic diagram of a pulse electric field energy packet that can be provided by a pulse ablation device in a second possible embodiment;
Figure 18 is a schematic diagram of a pulse electric field energy packet that can be provided by a pulse ablation device according to the third embodiment;
Figure 19 is a schematic diagram of a pulse electric field energy packet that can be provided by a pulse ablation device in a fourth possible embodiment;
Figure 20 is a structural diagram of the electrode assembly in an expanded state according to the fourth embodiment;
Figure 21 is a structural diagram of the electrode assembly in an expanded state according to the fifth embodiment;
Figure 22 is a structural diagram of the electrode assembly in an expanded state according to the sixth embodiment;
Figure 23 is a structural diagram of the electrode assembly in an expanded state according to the seventh embodiment.

### Reference numbers:

10-electrode assembly; 20-tube body; 30-handle assembly; 40-position sensing device; 50-pulse group; 101-cap; 102-electrode; 103-connecting tube; 104-rigid electrode; 301-wheel mechanism; 302-connector assembly; 501-high-frequency and high-voltage sub-pulse; 502-low-frequency and low-voltage sub-pulse; 1021-extended segment; 1022-first converged end; 1023-second converged end; 201-traction structure; 202-insulating tube; 203-channel tube; 204-outer catheter; 205-stress tube; 1011-end cap; 1012-electrode ring; 1011a-end portion; 1011b-connecting post.

### DETAILED DESCRIPTION

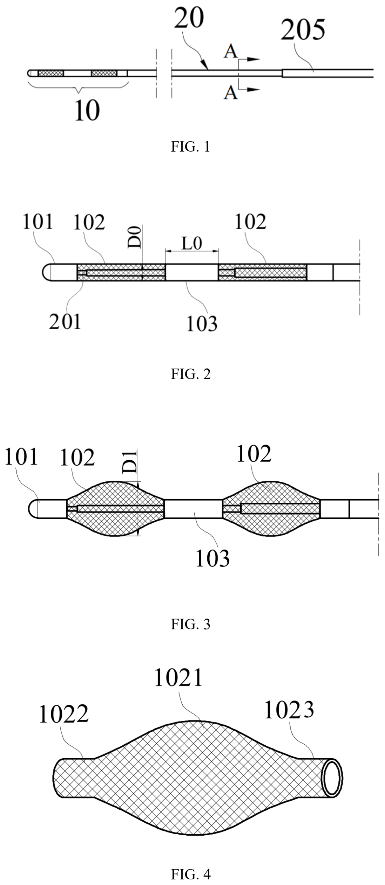

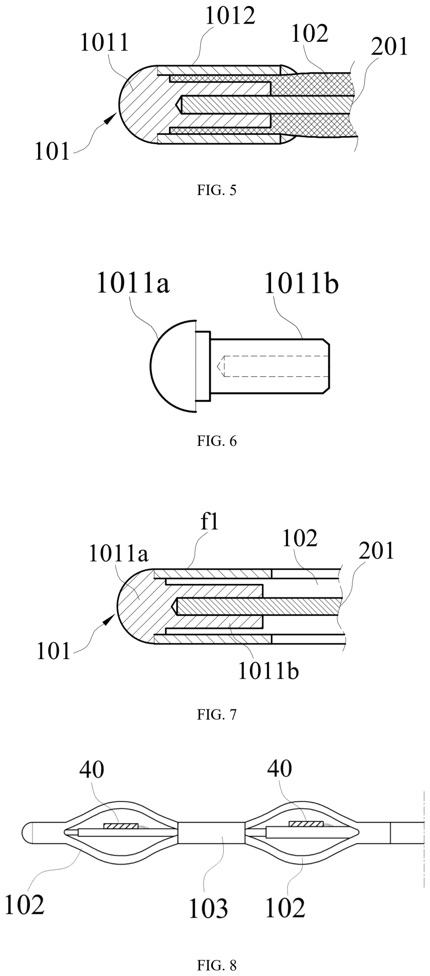

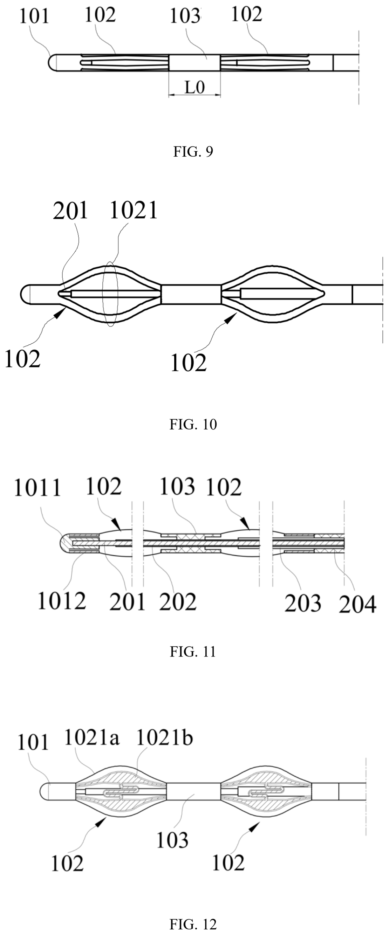

Referring to Figures 1-23 attached to the embodiments of the present application, it provides a clear and complete description of the technical solutions in these embodiments. It is apparent that the described embodiments are merely a part of the present application, not all of them. Based on the embodiments of the present application, all other embodiments obtained by ordinary technicians in the field without creative efforts belong to the scope of protection of the present application.

It should be noted that the terms "first," "second," etc., in the specification, claims, and accompanying drawings of the present application are configured to distinguish similar objects and do not necessarily describe a specific order or sequence. It should be understood that such used numerals can be interchanged in appropriate circumstances, so that the embodiments of the present application described here can be implemented in a sequence other than that illustrated or described here. Further, the terms "comprise" and "have" and any variations of them are intended to cover non-exclusive inclusion.

As shown in Figures 1-23, the embodiment provides a flexible electrode pulse ablation catheter, which comprises: a tube body 20 having a distal end and a proximal end disposed opposite to each other, wherein the tube body 20 comprises a traction structure 201 and a delivery tube assembly sleeved on the traction structure 201; an electrode assembly 10 disposed at the distal end of the tube body 20, wherein the electrode assembly 10 comprises at least one connecting tube 103 and at least two electrodes 102. Wherein adjacent two electrodes 102 are fixedly connected by the connecting tube 103, and the electrode 102 is a tubular structure with a hollow cavity inside; one end of the traction structure 201 passes through the delivery tube assembly and extends into the electrode assembly 10, and is fixedly connected to one end of the electrode 102 disposed near the distal end of the tube body 20; the traction structure 201 can reciprocate under external force along the axial direction of the delivery tube assembly to drive the opposite ends of the same electrode 102 to move towards or away from each other until the tubular structure contracts or expands.

The electrode assembly 10, traction structure 201, and delivery tube assembly in this embodiment cooperate with each other to achieve synchronous contraction or expansion of at least two electrodes 102 in the electrode assembly 10, ensuring that the flexible electrode pulse ablation catheter can bend and expand along the bronchial lumen, flexibly adjust the morphology of the electrodes 102 to adapt to the lesion structure, and achieve larger electrode-to-lesion contact area; and the electrode assembly 10 comprises at least two electrodes 102 spaced apart, which can reduce the stiffness of the electrodes 102, improve the ductility of the electrodes 102, increase the electrode coverage area, and enhance the flexibility of the electrode assembly 10, ensuring that the electrode assembly 10 can enter lesions with larger bending angles, and adjacent two electrodes 102 are fixedly connected by the connecting tube 103 to achieve synchronous contraction or expansion of adjacent two electrodes 102, which can further improve the flexibility of the electrode assembly 10, achieve larger electrode-to-lesion contact area, realize the delivery and ablation of lesions at large peripheral angles, ensure ablation effect, and improve ablation success rate.

In this embodiment, the flexible electrode pulse ablation catheter is mainly used for lung tumors, especially for lung tumors with large bending angles that are difficult to observe or access. The flexible electrode pulse ablation catheter of the flexible electrode pulse ablation catheter is inserted into the lung lesion tissue through a bronchoscope, and the outer surface of at least two electrodes 102 arranged at intervals is attached to the lesion tissue to achieve ablation of the lung lesion tissue by the pulse formed between the at least two electrodes 102; the problems of difficult bending and deformation of the ablation catheter, low electrode 102 coverage rate, resulting in difficult electrode 102 delivery and narrow ablation range can be solved by setting the above-mentioned flexible electrode pulse ablation catheter.

Preferably, the electrode assembly 10 comprises two electrodes 102 spaced apart, and the pulse formed between the two spaced-apart electrodes 102 can emit pulses with microsecond-level widths to the lung lesion tissue, thereby damaging the stability of the cell membrane surface of the lung lesion tissue, causing multiple hydrophilic micropores to appear on the surface of the lung lesion tissue cells, disrupting the steady state of the lung lesion tissue cells, and causing the death of the lung lesion tissue cells, thereby achieving the therapeutic effect of pulse ablation of the lung lesion tissue.

It can be understood that different lung lesion tissue cells have different destruction thresholds. By controlling the size of the pulse energy emitted by the electrode assembly 10, the pulses emitted by at least two electrodes 102 can be precisely targeted to the lesion location, and pulse ablation therapy has tissue cell selectivity; the pulse only targets lung lesion tissue cells for pulse ablation therapy without damaging other tissue cells by controlling at least two electrodes 102 to emit pulse energy corresponding to lung lesion tissue cells.

In this embodiment, the outer diameter of the electrode 102 can be extended and deformed to increase the contact area with the lesion; the conductor portion in the electrode 102 preferably comprises materials with excellent biocompatibility and corrosion resistance, such as medical stainless steel, nickel-titanium alloy, platinum-iridium alloy, gold, silver, platinum, titanium, tungsten, palladium, and other materials.

In this embodiment, the electrode assembly 10 comprises one connecting tube 103 and two electrodes 102. The first converged end 1022 of the first electrode 102 is fixedly connected to the first end of the traction structure 201, and the second converged end 1023 of the first electrode 102 is fixedly connected to the first end of the connecting tube 103. The second end of the connecting tube 103 is fixedly connected to the first converged end 1022 of the second electrode 102, and the second converged end 1023 of the second electrode 102 is fixedly connected to the first end of the delivery tube assembly. The second end of the delivery tube assembly can be fixedly connected to the housing of the handle assembly 30, and the second end of the traction structure 201 can be fixedly connected to the wheel mechanism 301 of the handle assembly 30. This ensures that when the traction structure 201 is driven by the wheel mechanism 301, the traction structure 201 reciprocates relative to the delivery tube assembly, thereby driving the first converged end 1022 and the second converged end 1023 of the same electrode 102 to move towards or away from each other, achieving contraction or expansion of the electrode 102.

In some other possible embodiments, the electrode assembly 10 comprises two connecting tubes 103 and three electrodes 102. The first electrode 102, the first connecting tube 103, the second electrode 102, the second connecting tube 103, and the third electrode 102 are sequentially fixedly connected along the axial direction of the traction structure 201. Compared to spacing two electrodes 102 apart, spacing three electrodes 102 apart can further improve the ductility of the electrode assembly 10 and simultaneously enhance its flexibility, enabling a larger area of the electrodes 102 to adhere to the lesion and facilitating delivery and ablation of lesions at large angles around the lesion, ensuring ablation effectiveness.

In other possible embodiments, the electrode assembly 10 comprises three connecting tubes 103 and four electrodes 102. the first electrode 102, the first connecting tube 103, the second electrode 102, the second connecting tube 103, the third electrode 102, the third connecting tube 103, and the fourth electrode 102 are sequentially fixedly connected along the axial direction of the traction structure 201. Compared to spacing three electrodes 102 apart, spacing four electrodes 102 apart can further improve the ductility of the electrode assembly 10 and simultaneously enhance its flexibility, enabling an even larger area of the electrodes 102 to adhere to the lesion and facilitating delivery and ablation of lesions at large angles around the lesion, ensuring ablation effectiveness.

In some possible embodiments, the electrode 102 comprises at least one of the following features: when the electrode 102 is in a contracted state, the maximum diameter of the electrode 102 is between 0.5mm-5mm; when the electrode 102 is in an expanded state, the maximum diameter of the electrode 102 is between 1.5mm-20mm. By setting the relevant dimensions of the electrode within the above ranges, it ensures that the electrode 102 is easy to move within the bronchus and can guarantee the contact area between the electrode 102 and the bronchus, achieving maximum adhesion of the electrode 102 to the lesion and a larger radial ablation range, thereby ensuring ablation effectiveness.

In this embodiment, when the electrode 102 is in a contracted state, the maximum diameter of the electrode 102 is within ranges such as 0.5mm-2mm, 0.5mm-5mm, 1.0mm-2.0mm, 1.5mm-2.0mm, 1.0mm-3mm, 2.0mm-2.6mm, 2.3mm-2.6mm, 2.5mm-3.0mm, 2mm-5mm, or 3mm-5mm, etc.; when the electrode 102 is in an expanded state, the maximum diameter of the electrode 102 is within ranges such as 1.5mm-5mm, 1.5mm-8mm, 1.5mm-20mm, 4mm-6mm, 5mm-10mm, 6mm-10mm, 8mm-10mm, 10mm-12mm, 10mm-15mm, or 18mm-20mm, etc.

In some possible embodiments, when the electrode 102 is in a contracted state, the cross-section of the electrode 102 is cylindrical; when the electrode 102 is in an expanded state, the cross-section of the electrode 102 is elliptical. Such settings ensure that during delivery, the volume of the electrode 102 is as small as possible and the shape is a cylindrical structure conducive to delivery, guaranteeing delivery speed. Meanwhile, when the electrode 102 is in an expanded state, the elliptical cross-section of the electrode 102 increases the contact area between the electrode 102 and the lesion, ensuring ablation effectiveness.

In this embodiment, the outer surface of the electrode 102 is a conductive surface. During the ablation process, as the traction structure 201 drives the electrode 102 to contract or expand, the traction structure 201 changes the axial distance of the extended segment 1021, thereby adjusting the diameter of the extended segment 1021. This adapts to the wall-adhering requirements of different lesions during ablation, enhancing adaptability while ensuring ablation effectiveness.

In some possible embodiments, the electrode assembly 10 comprises at least one of the following features: the spacing between adjacent two electrodes 102 is between 5mm-20mm; the length of the electrode assembly 10 is between 10mm-40mm. It ensures the ductility of the electrode assembly 10 and guarantees its ablation effectiveness by setting the spacing between adjacent two electrodes 102 within the above range.

In this embodiment, the number of electrodes 102 comprised in the electrode assembly 10 is not limited, as long as the spacing between adjacent two electrodes 102 meets the above range and the length of the electrode assembly 10 conforms to the above range.

In this embodiment, the spacing between adjacent two electrodes 102 is within ranges such as Smm-10mm, 5mm-15mm, 5mm-20mm, 10mm-15mm, 10mm-20mm, or 15mm-20mm, etc.; the length of the electrode assembly 10 is within ranges such as 10mm-20mm, 10mm-30mm, 15mm-40mm, 20mm-40mm, 20mm-35mm, or 30mm-40mm, etc.

In some possible embodiments, the adjacent two electrodes 102 have opposite polarities. This setup ensures that the ablation range is less affected by tissue impedance, concentrating the ablation energy around the electrodes 102, thereby increasing the ablation range of the electrode assembly 10. It avoids the narrow ablation range of a single electrode 102, which can be easily influenced by air in the lungs, leading to incomplete ablation. More importantly, the pulse energy range generated by the bipolar electrodes 102 is controllable, ensuring that the damage range of the pulse energy is also controllable. This achieves the technical effects of minimal damage to adjacent nerves and blood vessels and fewer postoperative complications.

In this embodiment, the electrode assembly 10 comprises two electrodes 102, with the first electrode 102 electrically connected to the negative pole and the second electrode 102 electrically connected to the positive pole, achieving opposite polarities for the two electrodes 102 and enabling bipolar ablation.

In some possible embodiments, the tubular structure has a first converged end 1022 and a second converged end 1023 at its two ends, respectively. The first converged end 1022 and the second converged end 1023 are arranged along the axial direction of the delivery tube assembly. The first converged end 1022 is disposed near the distal end of the tube body 20, while the second converged end 1023 is disposed near the proximal end of the tube body 20. One end of the delivery tube assembly is fixedly connected to the second converged end 1023 of the electrode 102 disposed near the proximal end of the tube body 20. One end of the traction structure 201 passes through the delivery tube assembly and extends into the electrode assembly 10, and is fixedly connected to the first converged end 1022 of the electrode 102 disposed near the distal end of the tube body 20. One end of the traction structure 201 is connected to the first converged end 1022 of the electrode 102 near the distal end of the tube body 20 after passing through the delivery tube assembly, and the delivery tube assembly is fixedly connected to the second converged end 1023 of the electrode 102 near the proximal end of the tube body 20, with adjacent electrodes 102 fixedly connected by a connecting tube 103. This setup ensures that each electrode 102 in the electrode assembly 10 can synchronously contract or expand. This enables the electrode assembly 10 to bend and expand according to the bronchial lumen, flexibly adjusting the shape of the electrodes 102 to adapt to the lesion structure, achieving greater electrode contact with the lesion and thereby improving the success rate of pulsed ablation.

In this embodiment, the lengths and shapes of the first converged end 1022 and the second converged end 1023 are not limited, as long as the diameters of the first converged end 1022 and the second converged end 1023 are not greater than the diameter of the extended segment 1021 in the contracted state.

In this embodiment, the first converged end 1022 and the second converged end 1023 are made of materials that are not prone to deformation, and both are annular structures.

In this embodiment, the first converged end 1022, the extended segment 1021, and the second converged end 1023 are integrally formed or separately formed, depending on the actual situation, and are not limited here.

In this embodiment, the electrode 102 can be a tubular structure with an internal cavity composed of a grid array structure, a simply supported beam structure, or a balloon structure.

The electrode assembly 10 is configured to deliver nanosecond and/or microsecond pulsed electric field energy to the target lesion for irreversible electroporation ablation. To enhance the depth and safety of bronchial interventional ablation and reduce tumor recurrence rates, the electrode assembly 10 is configured so that at least two adjacent electrodes 102 can contact the lesion, enabling bipolar ablation. That is, the pulsed electric field is transmitted through adjacent electrodes of opposite polarity. Compared to monopolar ablation, bipolar ablation achieves a greater ablation depth with the same pulse energy parameters and has a smaller impact on muscle tremors. Additionally, the electrode assembly 10 adopts a "island-bridge" flexible structural design with adjacent electrodes 102 spaced apart, which further reduces the stiffness of the electrodes 102 compared to conventional non-extendable ring electrodes or needle electrode structures, enhances the ductility of the electrodes 102, and increases the electrode coverage area. The electrode assembly 10 is composed of grid arrays, simply supported beam structures, or balloon structures, avoiding the adverse effects of increased electrode 102 length or diameter on delivery and adherence, and achieving optimal lung peripheral bronchial delivery and conformal ablation.

In some possible embodiments, electrode 102 is configured as a tubular structure woven from metallic wires. Such a design ensures the connection stability of electrode 102 and guarantees maximum contact area with the lesion regardless of the rotation angle of electrode 102, further enhancing ablation effectiveness. Meanwhile, it mitigates adverse effects on delivery and abutment due to increased length or diameter of electrode 102, achieving superior delivery outcomes and conformal ablation.

In this embodiment, electrode 102 adopts a grid array structure woven from metallic wires, utilizing weaving patterns such as one-over-one, one-over-two, or two-over-two. The metallic wires preferably consist of metals like medical-grade stainless steel, nickel-titanium alloy, platinum-iridium alloy, gold, silver, etc. The multiple metallic wires on electrode 102 form a mesh conductive surface for transmitting pulse energy, while possessing excellent deformation capability. Based on the spaced arrangement of electrode 102 within electrode assembly 10, this further enhances the deformation capacity of electrode 102, making the flexible electrode pulsed ablation catheter easily bendable. This allows for flexible adjustment of electrode 102's morphology to adapt to lesion structures, achieving greater electrode-to-lesion abutment area and thus improving the success rate of pulsed ablation.

In this embodiment, electrode 102 woven from metallic wires sequentially comprises a first converging end 1022, an extended segment 1021, and a second converging end 1023, all integrally formed. Under the action of traction structure 201, the first converging end 1022 and the second converging end 1023 can move towards each other to compress the extended segment 1021, thereby increasing its diameter and enhancing abutment area with the lesion. Alternatively, they can move apart to compress the extended segment 1021, reducing its diameter and facilitating electrode 102 delivery.

In some possible embodiments, electrode 102 further comprises an extended segment 1021, with its ends fixedly connected to the first converging end 1022 and the second converging end 1023, respectively. The extended segment 1021 comprises at least two first connecting members, spaced circumferentially around the delivery tube assembly. By spacing the at least two first connecting members circumferentially around the delivery tube assembly, it not only ensures contact area between the extended segment 1021 and the lesion but also enhances the extended segment's ductility, thus increasing electrode 102's flexibility. This enables flexible adjustment of electrode 102's morphology to adapt to lesion structures, achieving greater electrode-to-lesion abutment area and improving pulsed ablation success rates. It also mitigates adverse effects on delivery and abutment due to increased electrode length or diameter, achieving superior delivery outcomes and conformal ablation.

In some possible embodiments, the surface slope of the first connecting member gradually increases from small to large and then decreases from large to small, forming an arched structure along the direction from the first converging end 1022 to the second converging end 1023. Configuring the first connecting member as an arched structure ensures that electrode 102 can undergo bending deformation with slight action from traction structure 201, enhancing electrode 102's responsiveness and improving the flexible electrode pulsed ablation catheter's speed. This saves ablation duration and enhances user experience.

In this embodiment, the first connecting member is a simply supported beam. Initially, when electrode 102 is in a contracted state, the simply supported beam closes to form a micro-arched structure. When electrode 102 is in an expanded state, the simply supported beam bends along its thickness direction.

In this embodiment, the extended segment 1021 of the simply supported beam is initially configured in an arched structure to achieve the desired electrode 102 expansion morphology. In some embodiments, the arched structure is achieved through pre-compression assembly, where electrode 102 is moved proximally via traction structure 201 for pre-compressed installation, causing mild bending deformation of the simply supported beam's extended segment 1021. For full expansion, the traction structure 201 is moved proximally via the operating handle assembly until an ellipsoidal surface expansion is achieved. In other embodiments, the arched structure can be achieved through preset shaping, where the simply supported beam is fixed on a shaping fixture and pre-bent and shaped through high-temperature heat treatment.

In some possible embodiments, when electrode 102 is in a compressed state, the length direction of the first connecting member is parallel to the axial direction of the delivery tube assembly, and the thickness direction of the first connecting member passes through the axis of the delivery tube assembly. The first connecting member comprises at least one of the following features: the thickness of the first connecting member is much smaller than its width; the width of the first connecting member is much smaller than its length; the number of first connecting members ranges from 2 to 14. These constraints on the first connecting member ensure its ductility, thereby increasing electrode 102's flexibility. This enables flexible adjustment of electrode 102's morphology to adapt to lesion structures, achieving greater electrode-to-lesion abutment area and improving pulsed ablation success rates.

In some possible embodiments, an extended segment 1021 is further comprised, with both ends of the extended segment 1021 fixedly connected to a first converged end 1022 and a second converged end 1023, respectively. The extended segment 1021 comprises a balloon membrane and an electrode membrane arranged sequentially from the inside out. The interior of the balloon membrane has a sealed cavity that can communicate with a delivery tube assembly, allowing liquid to enter or exit the sealed cavity through the delivery tube assembly to cause the balloon membrane to contract or expand. By incorporating the expandable extended segment 1021 between the first converged end 1022 and the second converged end 1023, the electrode 102 can ensure the area of contact with the lesion while avoiding adverse effects on delivery and contact due to increased length or diameter of the electrode 102, achieving better delivery effectiveness and conformal ablation.

In this embodiment, the balloon membrane is a compliant or semi-compliant membrane.

In some possible embodiments, the electrode membrane comprises at least two second connectors spaced apart around the circumference of the balloon membrane. By arranging at least two second connectors, it is possible to reduce the strength of the electrode membrane while ensuring the contact area, thereby improving the electrode membrane's expansibility and enabling it to deform easily, so that the electrode membrane can flexibly adjust the shape of the electrode 102 to adapt to the lesion structure, achieving greater contact area between the electrode 102 and the lesion and thus enhancing the success rate of pulsed ablation.

In some possible embodiments, the electrode membrane comprises at least one of the following features: the thickness of the electrode membrane is 10µm to 100µm; the electrode membrane has a multilayer membrane structure. Setting the thickness of the electrode membrane within the above range ensures its limitation on the balloon membrane disposed therein while also ensuring that the electrode membrane is prone to deformation under external force, thereby guaranteeing the contact area between the electrode membrane and the lesion and ensuring the ablation effect.

In some possible embodiments, when the electrode 102 is in a compressed state, the length direction of the second connectors is parallel to the axial direction of the delivery tube assembly, and the thickness direction of the second connectors passes through the axis of the delivery tube assembly. The second connectors satisfy at least one of the following features: the thickness of the second connectors is much smaller than their width; the width of the second connectors is much smaller than their length; the number of second connectors is 2 to 10. The above restrictions on the second connectors can ensure their expansibility, thereby increasing the flexibility of the electrode 102. This allows for flexible adjustment of the electrode 102's shape to adapt to the lesion structure, achieving greater contact area between the electrode 102 and the lesion and thus enhancing the success rate of pulsed ablation.

In this embodiment, the electrode membrane 1021b has a multilayer membrane structure; the outer surface of the electrode membrane 1021b facing away from the balloon membrane 1021a is made of a conductive material, preferably a biocompatible material such as gold, platinum, titanium, tungsten, palladium, silver, etc.; the inner surface of the electrode membrane 1021b facing towards the balloon membrane 1021a serves as a flexible substrate, preferably made of a biocompatible material such as polyurethane or polyimide; the multilayer structure of the electrode membrane 1021b is manufactured through semiconductor processes such as magnetron sputtering, electroplating, chemical etching, etc., with the overall thickness controlled between 10µm and 100µm; the electrode membrane 1021b has a flexible microstructure, such as transition connections made through serpentine conductors, for achieving conformal connection and folded compression with the balloon membrane 1021a; during delivery, the electrode membrane 1021b collapses with the evacuation of the balloon membrane 1021a, assuming a coiled and folded state with a maximum diameter D0 of φ0.5 mm to φ5 mm; the extended segment 1021 of the electrode 102 expands the balloon membrane 1021a through internal fluid injection during operation, causing the electrode membrane 1021b to unfold to a maximum diameter D1 of φ1.5 mm to φ20 mm.

Further, a traction structure 201 is provided running through the interior of the electrode 102; an insulating tube 202 is movably sleeved over the upper portion of the traction structure 201, and a channel tube 203 is movably sleeved over the outer portion of the insulating tube 202. A liquid injection channel is provided between the traction structure 201 and the insulating tube 202 for infusion and expansion of the balloon membrane 1021a on the electrode 102 near the distal end of the tube body 20. Another liquid injection channel is provided between the insulating tube 202 and the channel tube 203 for infusion and expansion of the balloon membrane 1021a on the electrode 102 near the proximal end of the tube body 20.

In some possible embodiments, the electrode assembly 10 further comprises a cap 101 and a rigid electrode 104; the first end of the cap 101 is provided with an arcuate surface, and the second end of the cap 101 is provided with a traction structure mounting position and a converged end mounting position; the traction structure mounting position is configured for fixed connection with one end of the traction structure 201; the first end of the rigid electrode 104 is fixedly connected to the second converged end 1023 of the electrode 102 disposed near the proximal end of the tube body 20, the second end of the rigid electrode 104 is fixedly connected to the tube body 20, and the converged end mounting position is configured for fixed connection with the first converged end 1022 of the electrode 102 disposed near the distal end of the tube body 20. By providing the cap 101, it is possible to fix the electrode 102 near the distal end of the tube body 20, and simultaneously, by providing the cap 101 with an arcuate surface, it can avoid causing injury to the human body during movement of the cap 101, thereby improving the safety of the flexible electrode pulse ablation catheter.

Specifically, both the cap 101 and the rigid electrode 104 are made of radiopaque materials. The cap 101 can function as a rigid electrode, and the polarity of the cap 101 is the same as that of its adjacent electrode 102. The polarity of the rigid electrode 104 is the same as that of its adjacent electrode 102.

In some possible embodiments, the length of the cap 101 is less than the length of the electrode 102, and the length of the rigid electrode 104 is also less than the length of the electrode 102.

Preferably, the length of the cap 101 is 30%-60% of the length of the electrode 102, and the length of the rigid electrode 104 is 30%-60% of the length of the electrode 102.

In some possible embodiments, both the cap 101 and the rigid electrode 104 can be considered equivalent to the electrode 102, meaning at least one of the cap 101 and the rigid electrode 104 can function as an electrode 102. In cases where the electrode assembly 10 comprises two electrodes 102, the cap 101 or the rigid electrode 104 can serve as an electrode 102, thereby enabling the electrode assembly 10 to comprise two electrodes 102.

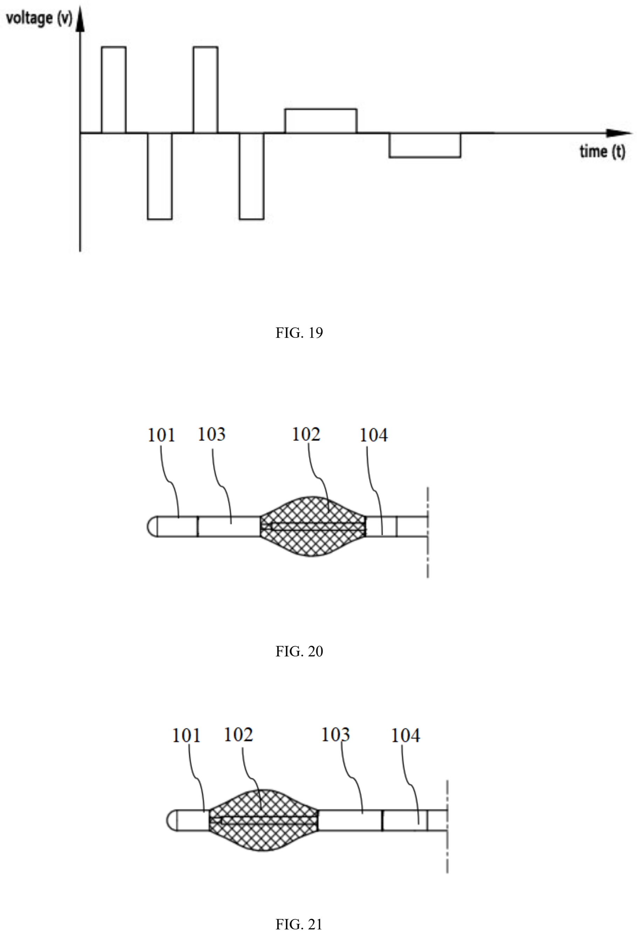

Referring to Figure 20, the electrode assembly 10 at this time comprises one electrode 102 in an expanded state. In a contracted state, the diameter of the electrode 102 is smaller than when it is in the expanded state. the electrode assembly 10 comprises a cap 101, a connecting tube 103, an electrode 102, and a rigid electrode 104 along the length direction of the electrode assembly 10. At this time, the cap 101 functions as a rigid electrode and is equivalent to an electrode 102. The cap 101 and the electrode 102 are spaced apart by the connecting tube 103. The polarity of the cap 101 is opposite to that of the electrode 102, while the polarity of the electrode 102 is the same as that of the rigid electrode 104.

Referring to Figure 21, the electrode assembly 10 at this time comprises one electrode 102 in an expanded state. In a contracted state, the diameter of the electrode 102 is smaller than when it is in the expanded state. The electrode assembly 10 comprises a cap 101, an electrode 102, a connecting tube 103, and a rigid electrode 104 along the length direction of the electrode assembly 10. At this time, the cap 101 is equivalent to a rigid electrode and functions as such. The electrode 102 and the rigid electrode 104 are spaced apart by the connecting tube 103. The rigid electrode 104 is equivalent to an electrode 102. The polarity of the electrode 102 is opposite to that of the rigid electrode 104, while the polarity of the cap 101 is the same as that of the electrode 102.

In this embodiment, the structures of adjacent two electrodes 102 can be the same or different, which is not limited herein. Preferably, the structures of adjacent two electrodes 102 are the same. In cases where the structures of adjacent two electrodes 102 are different, the electrode 102 can be a tubular structure woven from metal wires, or can have an extended segment 1021 comprising at least two first connecting members spaced apart around the circumference of the delivery tube assembly, or can have an extended segment 1021 comprising a balloon membrane and an electrode membrane disposed from the inside out.

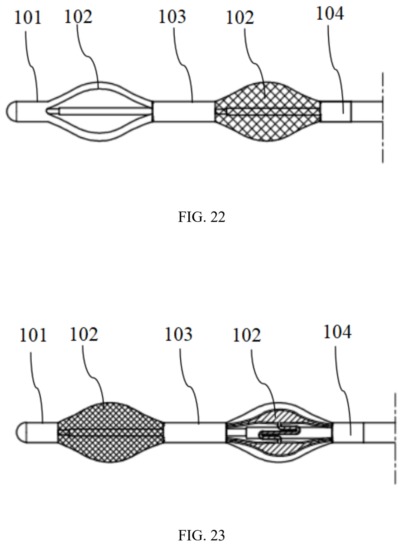

Referring to Figure 22, the electrode assembly 10 at this time comprises two electrodes 102, both of which are in an expanded state. In a contracted state, the diameter of the electrodes 102 is smaller than when they are in the expanded state. The electrode assembly 10 comprises a cap 101, a first electrode 102, a connecting tube 103, a second electrode 102, and a rigid electrode 104 along the length direction of the electrode assembly 10. At this time, the cap 101 functions as a rigid electrode and is equivalent to an electrode. The polarity of the cap 101 is the same as that of its adjacent electrode 102, and the polarity of the rigid electrode 104 is the same as that of its adjacent electrode 102. The first electrode 102 and the second electrode 102 are spaced apart by the connecting tube 103. The extended segment 1021 of the first electrode 102 comprises at least two first connecting members spaced apart around the circumference of the delivery tube assembly, while the second electrode 102 is a tubular structure woven from metal wires.

Referring to Figure 23, the electrode assembly 10 comprises two electrodes 102, both of which are in an expanded state. When the electrodes 102 are in a contracted state, their diameter is smaller compared to when they are in the expanded state. Along the length of the electrode assembly 10, it comprises a cap 101, a first electrode 102, a connecting tube 103, a second electrode 102, and a rigid electrode 104. At this time, the cap 101 functions equivalently to a rigid electrode and has the same polarity as its adjacent electrode 102, while the rigid electrode 104 has the same polarity as its adjacent electrode 102. The first electrode 102 and the second electrode 102 are spaced apart by the connecting tube 103. The first electrode 102 is a tubular structure woven from metallic wires, while the extended segment 1021 of the second electrode 102 comprises a balloon membrane and an electrode membrane disposed sequentially from the inside out.

In some possible embodiments, the cap 101 comprises an end cap 1011 and an electrode ring 1012; an arcuate surface is disposed at a first end of the end cap 1011, and the electrode ring 1012 is fixedly connected to a second end of the end cap 1011. After the electrode ring 1012 is connected to the second end of the end cap 1011, a converged end mounting position is formed between the electrode ring 1012 and the end cap 1011. A traction structure mounting position is disposed at the second end of the end cap 1011. By providing the cooperating end cap 1011 and electrode ring 1012, it facilitates the installation and removal of the electrode 102, reducing the preparation time for the flexible electrode pulse ablation catheter.

In this embodiment, the distal end of the end cap 1011 is an end portion 1011a, and the proximal end is a connecting post 1011b; the end cap 1011 and the electrode ring 1012 are fixedly connected by welding, clipping, adhering, or other methods; the end portion 1011a of the end cap 1011 is a semi-circular or conical structure, and a blind hole is provided within the connecting post 1011b of the end cap 1011, with a traction structure 201 fixedly connected thereto; the end cap 1011 and the electrode ring 1012 are made of conductive materials, preferably metals such as medical stainless steel, nickel-titanium alloy, platinum-iridium alloy, gold, silver, etc.

In this embodiment, the area within the connecting post 1011b where the blind hole is disposed is the traction structure mounting position, and the annular area formed between the electrode ring 1012 and the end cap 1011 is the converged end mounting position.

In some possible embodiments, the stiffness of the tube body 20 gradually decreases along the proximal end to the distal end of the tube body 20. By gradually decreasing the stiffness of the tube body 20, it ensures the support at the proximal end of the tube body 20 while enhancing the flexibility at the distal end, facilitating the electrode assembly 10 disposed at the distal end of the tube body 20 to move within lesions with greater curvature and improving the delivery rate of the electrode assembly 10.

In this embodiment, the tube body 20 is configured to deliver the electrode assembly 10 to the lesion via a bronchoscope channel.

In this embodiment, the tube body 20 comprises a traction structure 201 and a delivery tube assembly, and the stiffness of the delivery tube assembly gradually decreases along the proximal end to the distal end of the tube body 20.

Preferably, the stiffness of the outer catheter 204 gradually decreases along the proximal end to the distal end of the tube body 20.

In other possible embodiments, the delivery tube assembly comprises at least one of the following features: along the proximal end to the distal end of the tube body 20, the stiffness of the outer catheter 204 gradually decreases; along the proximal end to the distal end of the tube body 20, the stiffness of the insulating tube 202 gradually decreases; along the proximal end to the distal end of the tube body 20, the stiffness of the channel tube 203 gradually decreases; along the proximal end to the distal end of the tube body 20, the stiffness of the stress tube 205 gradually decreases.

In this embodiment, by gradually decreasing the hardness of the outer catheter 204, the channel tube 203, and the insulating tube 202, it is possible to maximize the support at the proximal end of the tube body 20 and maximize the flexibility at the distal end of the tube body 20, thereby increasing the maneuverability of the ablation catheter for pulsed electrode ablation and improving the success rate of ablation.

In some possible embodiments, the tube body 20 is made of a radiopaque material, enabling the position of the electrode 102 to be adjusted and confirmed under X-ray, which in turn enhances the accuracy of delivering the electrode assembly 10 to the lesion site.

In this embodiment, the material of the tube body 20 is at least one of polyetheramide, nylon, thermoplastic polyurethane, and polytetrafluoroethylene. The use of these materials ensures the delivery of the electrode 102 within curved or bifurcated lumens.

In some possible embodiments, an insulating tube 202, a channel tube 203, an outer catheter 204, and a stress tube 205 are sequentially arranged along the radial direction of the delivery tube assembly; a first end of the outer catheter 204 is fixedly connected to the electrode assembly 10, a second end of the outer catheter 204 can be fixedly connected to a handle assembly 30, and the stress tube 205 is disposed at the connection position between the outer catheter 204 and the handle assembly 30; part of the wires electrically connected to the electrode 102 can extend into the insulating tube 202, which is configured to isolate the wires electrically connected to adjacent electrodes 102 from each other; a gap exists between the inner wall of the channel tube 203 and the outer wall of the insulating tube 202, one end of the channel tube 203 communicates with the electrode assembly 10 to direct fluid to the location of the electrode assembly 10 through the channel tube 203; the insulating tube 202 in the delivery tube assembly is partially disposed, which can reduce the manufacturing cost of the delivery tube assembly while ensuring its flexibility; disposing the stress tube 205 only at the connection position between the outer catheter 204 and the handle assembly 30 can diffuse the stress at the connection and enhance the support at the proximal end of the delivery tube assembly.

In this embodiment, the traction structure 201 is a mandrel, with two electrodes 102 having mandrels running through their interiors. An upper portion of the mandrel may have an insulating tube 202 sleeved over it, and an outer portion of the insulating tube 202 may have a channel tube 203 movably sleeved over it. The outer side of the channel tube 203 is connected to the outer catheter 204, which is fixedly connected to the handle assembly 30 with a stress tube 205 sleeved over the connection. The stress at the connection between the outer catheter 204 and the handle assembly 30 can be uniformly diffused by disposing the stress tube 205, preventing the outer catheter 204 from breaking due to stress and thereby increasing its service life; preferably, the outer catheter 204 is a composite reinforced tube structure such as a braided tube or a coiled spring tube.

In this embodiment, when the electrode assembly 10 comprises two electrodes 102, the wire connected to the electrode 102 disposed closer to the distal end of the tube body 20 passes through the inside of the insulating tube 202 to the interior of the shell of the handle assembly 30 for electrical connection to a positive cable, while the wire connected to the electrode 102 disposed closer to the proximal end of the tube body 20 passes through the inside of the insulating tube 202 to the interior of the shell of the handle assembly 30 for electrical connection to a negative cable.

In some possible embodiments, It is further comprises at least one position sensing device 40, which is disposed inside the electrode 102 and fixedly mounted on the traction structure 201. The acquisition and communication of position, shape, and temperature information of the electrode 102 can be achieved by installing the position sensing device 40. With real-time electromagnetic positioning navigation guidance, the delivery planning of the electrode 102 can be realized, reducing the difficulty of electrode 102 placement and delivery, and further enhancing the ablation effect.

In this embodiment, at least one of the at least two electrodes 102 has a position sensing device 40 installed inside with insulation. The position sensing device 40 comprises but is not limited to a positioning sensor and/or a shape-sensing optical fiber and/or a temperature sensor, which is used for real-time monitoring of electrode 102 position data and/or electrode shape data and/or temperature information. When the position sensing device 40 is configured as a positioning sensor, at least one positioning sensor is arranged at the first converged end 1022 of the electrode 102 disposed closer to the distal end of the tube body 20, for collecting position and orientation data of the electrode 102. Based on the above position coordinate information, three-dimensional model reconstruction of the electrode 102 in space can be achieved. The positioning sensor is insulated and isolated from the electrode 102 by materials with high dielectric strength such as polyimide and tetrafluoroethylene. The positioning sensor is preferably a 5DOF or 6DOF electromagnetic positioning sensor, which is capable of real-time measurement of the spatial position coordinates of the electrode assembly 10, relaying position and orientation data to the ablation system interface for visualization, thereby enhancing user experience and market share.

The second aspect of the present application also protects a pulsed ablation device, which comprises a flexible electrode 102 pulsed ablation catheter with a handle assembly 30 as described above; a first end of the handle assembly 30 can be electrically connected to an ablation mechanism that emits ablation energy; one end of the delivery tube assembly is fixedly connected to a second end of the handle assembly 30, and one end of the traction structure 201 passes through the delivery tube assembly to be connected to the handle assembly 30 in a driving manner, enabling the handle assembly 30 to drive the traction structure 201 to reciprocate axially relative to the delivery tube assembly. The pulsed ablation device in this embodiment can be delivered via a bronchoscope for local bipolar mode ablation. The electrode 102 possesses bending ductility, high electrode coverage area, minimal impact from tissue impedance, slight impact on muscle tremors, and with the assistance of the electrode assembly 10 and electromagnetic positioning navigation, the position of the lesion and important structures can be determined in real-time, allowing accurate delivery of the ablation electrode assembly 10 into the tumor for ablation, thereby reducing postoperative complications and improving the success rate of irreversible electroporation ablation.

In some possible embodiments, the handle assembly 30 comprises at least two wires, a wheel mechanism 301, and a connector assembly 302; the wheel mechanism 301 is fixedly connected to one end of the traction structure 201, and the wheel mechanism 301 drives the traction structure 201 to reciprocate axially relative to the delivery tube assembly under external force; the first ends of the at least two wires are electrically connected to the connector assembly 302, which is located at the first end of the handle assembly 30 and electrically connected to the ablation mechanism; the second ends of the at least two wires are respectively electrically connected to the at least two electrodes 102, so that adjacent two electrodes 102 have opposite polarities. By arranging mutually cooperating wires, a wheel mechanism 301, and a connector assembly 302, it can be achieved that the electrodes 102 are electrically connected to the connector assembly 302 via the wires, and the electrodes 102 are fixedly connected to the wheel mechanism 301 via the traction structure 201. Driven by the traction structure 201, they reciprocate relative to the delivery tube assembly, and current can be supplied to the electrodes 102 via the connector assembly 302 to achieve ablation of the lesion.

In this embodiment, the handle assembly 30 further comprises a housing with an accommodating cavity inside. Part of the wheel mechanism 301 is disposed within the housing, while another part protrudes from the outer wall of the housing to facilitate operators in rotating the wheel mechanism 301. Part of the lead wire is disposed within the housing, and the connector assembly 302 is fixedly mounted on the housing and electrically connected to the lead wire disposed inside.

In this embodiment, the housing of the handle assembly 30 is electrically insulated from the electrode assembly 10 and is electrically connected to the ablation device via the connector assembly 302. The proximal end of the traction structure 201 is connected to the wheel mechanism 301 to achieve axial reciprocating movement, thereby facilitating the adjustment of the expansion or contraction of the extended segment 1021 of the electrode 102. The connector assembly 302 comprises pulse cables, pulse connectors, sensing cables and sensing connectors. The pulse cables and pulse connectors are electrically connected to the electrode assembly 10, while the sensing cables and sensing connectors are electrically connected to the position sensing device 40. The pulse connectors and sensing connectors are communicatively connected to the ablation device.

In this embodiment, the specific structure of the wheel mechanism 301 is not limited, as long as it ensures that when the wheel mechanism 310 rotates, it can drive the traction structure 201 to move relative to the delivery tube assembly.

In this embodiment, the housing of the handle assembly 30 has two oppositely disposed openings. One opening is fixedly connected to the delivery tube assembly, while the other opening is configured for threading out cables to install the connector assembly 302. After one opening is fixedly connected to the delivery tube assembly, a stress tube 205 is sleeved at the connection position between the housing and the delivery tube assembly.

In some possible embodiments, the housing of the handle assembly 30 is provided with a direction indicator and/or an extended length indicator. The direction indicator is configured to instruct the operator to rotate the wheel mechanism 301 in a first direction to extend the flexible electrode pulsed ablation catheter, or to rotate it in a second direction opposite to the first direction to shorten the catheter.

In this embodiment, the housing of the handle assembly 30 comprises a rotating area and a gripping area connected to each other. The wheel mechanism 301 is disposed in the rotating area, and the operator's hand is placed in the gripping area, which facilitates operation and enhances user experience, thereby improving market competitiveness.

The third aspect of the present application also protects a pulsed ablation device comprising an ablation mechanism and the pulsed ablation apparatus as described above. The ablation mechanism is configured to provide at least one pulse group 50 to the pulsed ablation apparatus. By setting the pulsed ablation apparatus and the ablation mechanism to cooperate with each other, the ablation electrode assembly 10 can be accurately delivered into the tumor for ablation, thereby reducing postoperative complications and improving the ablation success rate of irreversible electroporation. Meanwhile, the ablation mechanism can provide pulsed ablation energy with a pulse energy corresponding to the lung lesion tissue cells. This pulse only targets the lung lesion tissue cells for pulsed ablation treatment without damaging other tissue cells, thereby improving the compatibility range of the pulsed ablation device to a certain extent.

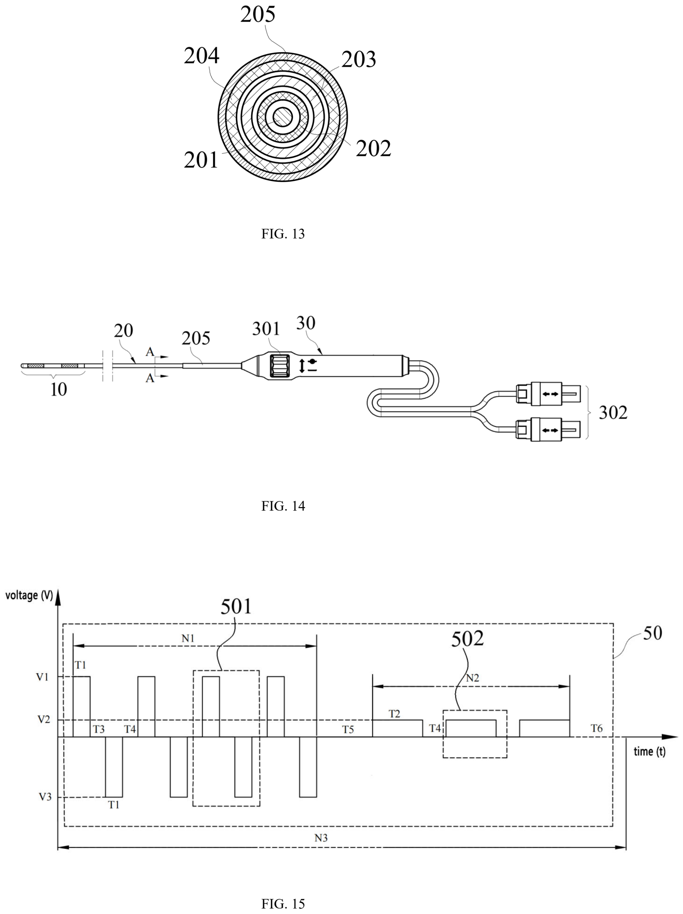

In this embodiment, in Figure 15, V1 represents the SHV voltage value; T1 represents the SHV pulse width; T3 represents the first pulse interval; T4 represents the second pulse interval; T5 represents the pulse repetition interval; T6 represents the inter-group period; N1 represents the number of SHV sub-pulses; V2 represents the LVV voltage value; T2 represents the LLV pulse width; N2 represents the number of LLV sub-pulses; and N3 represents the number of groups.

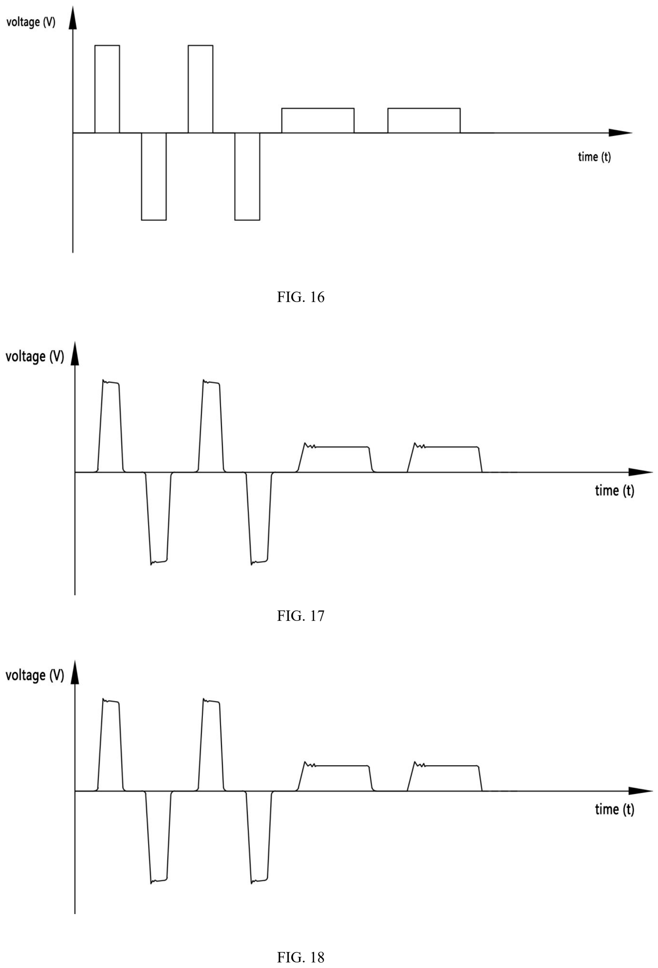

Figure 16 is a schematic diagram of a pulse electric field energy packet when the peak voltage plateau period is 100%, with high-frequency and high-voltage sub-pulses 501 being bipolar pulses and low-frequency and low-voltage sub-pulses 502 being unipolar pulses. Figure 17 is a schematic diagram of a pulse electric field energy packet when the peak voltage plateau period is 80%, with high-frequency and high-voltage sub-pulses 501 being bipolar pulses and low-frequency and low-voltage sub-pulses 502 being unipolar pulses. Figure 18 is a schematic diagram of a pulse electric field energy packet when the peak voltage plateau period is 80%, with high-frequency and high-voltage sub-pulses 501 being bipolar pulses and low-frequency and low-voltage sub-pulses 502 being bipolar pulses. Figure 19 is a schematic diagram of a pulse electric field energy packet when the peak voltage plateau period is 100%, with high-frequency and high-voltage sub-pulses 501 being bipolar pulses and low-frequency and low-voltage sub-pulses 502 being bipolar pulses.

In some possible embodiments, the pulse group 50 comprises at least one high-frequency and high-voltage sub-pulse 501 and/or one low-frequency and low-voltage sub-pulse 502. The high-frequency and high-voltage sub-pulse 501 is a bipolar pulse, and the low-frequency and low-voltage sub-pulse 502 is a unipolar pulse or a bipolar pulse. Among them, a bipolar pulse comprises positive and negative pulse amplitudes. By incorporating the high-frequency and high-voltage sub-pulse 501 and/or the low-frequency and low-voltage sub-pulse 502, the coverage of the pulse group 50 can be enhanced, thereby increasing the ablation range achievable by the pulse ablation setup.

In some possible embodiments, the pulse group 50 comprises at least one of the following features: the voltage amplitude range of the bipolar pulse of the high-frequency and high-voltage sub-pulse 501 is 2000V-70000V; the pulse width of the bipolar pulse of the high-frequency and high-voltage sub-pulse 501 is 50ns-50000ns; the number of bipolar pulses of the high-frequency and high-voltage sub-pulse 501 is 1-5000; the amplitude voltage of the unipolar pulse and the bipolar pulse of the low-frequency and low-voltage sub-pulse 502 is 200V-3000V; the pulse width of the unipolar pulse and the bipolar pulse of the low-frequency and low-voltage sub-pulse 502 is 10µs-500µs; the number of unipolar pulses and bipolar pulses of the low-frequency and low-voltage sub-pulse 502 is 1-5000; the number of pulse groups 50 is 1-5000. By setting the above ranges, the pulse ablation device can stably output energy, ensuring its ablation effectiveness.

In this embodiment, the voltage amplitude range of the bipolar pulse of the high-frequency and high-voltage sub-pulse 501 can be 2000V-5000V, 2000V-10000V, 5000V-10000V, 10000V-30000V, 20000V-50000V, or 50000V-70000V, etc.; the pulse width of the bipolar pulse of the high-frequency and high-voltage sub-pulse 501 can be 50ns-500ns, 50ns-5000ns, 100ns-5000ns, 2000ns-50000ns, 3000ns-40000ns, 20000ns-40000ns, 30000ns-50000ns, or 40000ns-50000ns, etc.; the number of bipolar pulses of the high-frequency and high-voltage sub-pulse 501 can be 1-100, 100-500, 500-2000, 500-5000, 1000-5000, 2000-3000, 3000-4000, or 4000-5000, etc.; the amplitude voltage of the unipolar pulse and the bipolar pulse of the low-frequency and low-voltage sub-pulse 502 can be 200V-300V, 200V-500V, 200V-1000V, 500V-1000V, 500V-3000V, 1000V-2000V, 1000V-3000V, or 2000V-3000V, etc.; the pulse width of the unipolar pulse and the bipolar pulse of the low-frequency and low-voltage sub-pulse 502 can be 10µs-50µs, 10µs-100µs, 10µs-500µs, 50µs-500µs, 100µs-200µs, 100µs-500µs, 200µs-500µs, or 300µs-500µs, etc.; the number of unipolar pulses and bipolar pulses of the low-frequency and low-voltage sub-pulse 502 can be 1-100, 1-500, 100-500, 100-1000, 100-5000, 500-1000, 500-2000, 500-5000, 1000-5000, 3000-5000, or 4000-5000, etc.; the number of pulse groups 50 can be 1-5000, 1-100, 1-500, 100-500, 100-1000, 100-5000, 500-1000, 500-2000, 500-5000, 1000-5000, 3000-5000, or 4000-5000, etc.

In this embodiment, the bipolar pulse of the high-frequency and high-voltage sub-pulse 501 consists of positive and negative pulse amplitudes, with a preferred voltage amplitude range of 2000V to 70000V; the pulse width of the bipolar pulse is preferably 50ns to 50000ns; a first pulse interval is provided between the positive and negative pulses of the bipolar pulse; a second pulse interval is provided between bipolar pulses; optionally, the number of bipolar pulses is preferably 1 to 5000.

Optionally, the amplitude voltage of the unipolar pulse and the bipolar pulse of the low-frequency and low-voltage sub-pulse 502 is preferably 200V to 3000V; the pulse width of the unipolar pulse and the bipolar pulse is preferably 10µs to 500µs; a second pulse interval is provided between unipolar pulses; an inter-group period is provided between pulse groups; the number of unipolar pulses and bipolar pulses is preferably 1 to 5000; the number of pulse groups is preferably 1 to 5000; a pulse repetition interval is provided between bipolar and unipolar pulses; an inter-group period is provided between pulse groups.

Optionally, unipolar and bipolar pulses exhibit rising edges or falling edges, or both, or the widths of the rising edges and falling edges are close to the pulse width; the rising and falling edge times of sub-pulses range from 0 to 500ns, and the length of the peak voltage plateau period of a single sub-pulse accounts for 0 to 100% of the pulse width. Figure 16 illustrates the pulse electric field energy packet when the length of the peak voltage plateau period of a single sub-pulse accounts for 100% of the pulse width, and Figure 17 illustrates the pulse electric field energy packet when the length of the peak voltage plateau period of a single sub-pulse accounts for 80% of the pulse width.

Working process of the pulse ablation Device is shown as follow. The electrode assembly 10 and tube body 20 are delivered to the lesion site via bronchoscopy. If the lesion location has been confirmed preoperatively, the electrode assembly 10 and tube body 20 can be inserted into the bronchoscope channel. Subsequently, electromagnetic localization technology is utilized to guide the tube body 20 to rapidly reach the lesion site along the optimal path, with the electrode assembly 10 being delivered into the lesion interior. Finally, CT imaging is configured to confirm the relative position of the electrode 102 to the lesion for ablation. Optionally, if the lesion location has not been confirmed preoperatively, delivery can be facilitated with a guiding sheath. Electromagnetic navigation is employed to advance the compatible guiding sheath near the lesion, followed by withdrawing the tube body 20. Further confirmation of the lesion location is achieved through radial ultrasound miniprobes and biopsy.

After pathological biopsy confirms the lesion location, the electrode assembly 10 is positioned in place. The rotation mechanism 301 of the handle assembly 30 is operated to appropriately deploy the electrodes 102. Once the tissue's electrical impedance is confirmed within an acceptable range, a low-dose pulse electric field energy packet is tested to observe muscle contraction and electrocardiographic synchronization before proceeding with ablation using normal parameters. Nanosecond and/or microsecond pulse electric field energy packets are applied to the target area, altering the permeability of organelles and/or cell membranes, leading to cell apoptosis or death. The synergistic ablation features of high-frequency and high-voltage sub-pulses and/or low-frequency and low-voltage sub-pulses enable the destruction of cancer cells while minimizing the impact on surrounding normal tissues. A CT scan is performed immediately after the ablation or 24 hours postoperatively to confirm that the real-time ablation range fully covers the tumor area. After confirming the absence of severe complications such as bleeding or pneumothorax, the flexible electrode pulse ablation device is removed to complete the procedure.

### Embodiment 1

In the figures 1-23, this embodiment provides a flexible electrode pulse ablation catheter comprising: a tube body 20 with a distal end and a proximal end opposite each other, wherein the tube body 20 comprises a traction structure 201 and a delivery tube assembly sleeved over the traction structure 201; an electrode assembly 10 disposed at the distal end of the tube body 20, the electrode assembly 10 comprising at least one connecting tube 103 and at least two electrodes 102, with adjacent electrodes 102 fixedly connected by the connecting tube 103, and the electrodes 102 being tubular structures with hollow interiors; one end of the traction structure 201 passes through the delivery tube assembly and extends into the electrode assembly 10, fixedly connected to one end of the electrode 102 positioned near the distal end of the tube body 20. The traction structure 201 can reciprocate under external force along the axial direction of the delivery tube assembly, causing the opposite ends of the same electrode 102 to move towards each other or away from each other, causing the tubular structure to contract or deploy.

In this embodiment, the flexible electrode pulse ablation catheter is primarily used for lung tumors, particularly those in lung areas with large curvature angles that are difficult to visualize or access. The flexible electrode pulse ablation catheter is inserted into the lung lesion tissue via bronchoscopy, with the outer surfaces of at least two electrodes 102 abutting against the lesion tissue to achieve ablation of the lung lesion tissue through pulses formed between the at least two electrodes 102. By configuring the flexible electrode pulse ablation catheter as described, it addresses issues such as the catheter's inflexibility, low electrode coverage, leading to difficulties in electrode delivery and limited ablation range.

Preferably, the electrode assembly 10 comprises two electrodes 102 arranged at an interval. The pulse formed between the two electrodes 102 spaced apart can emit microsecond-width pulses towards the lung lesion tissue, damaging the stability of the cell membrane surface of the lung lesion tissue and causing multiple hydrophilic micropores to appear on the surface of the lung lesion tissue cells. This disrupts the steady state of the lung lesion tissue cells, leading to their death and achieving the therapeutic effect of pulsed ablation of the lung lesion tissue.

In this embodiment, the tube body 20 comprises a traction structure 201 and a delivery tube assembly, with the stiffness of the delivery tube assembly gradually decreasing from the proximal end to the distal end of the tube body 20.

Preferably, the delivery tube assembly comprises at least one of the following features: the stiffness of the outer catheter 204 gradually decreases from the proximal end to the distal end of the tube body 20; the stiffness of the insulating tube 202 gradually decreases from the proximal end to the distal end of the tube body 20; the stiffness of the channel tube 203 gradually decreases from the proximal end to the distal end of the tube body 20; the stiffness of the stress tube 205 gradually decreases from the proximal end to the distal end of the tube body 20.

In this embodiment, the tube body 20 is made of a radiopaque material.

In this embodiment, the material of the tube body 20 is at least one of polyetheramide, nylon, thermoplastic polyurethane, and polytetrafluoroethylene. The above materials ensure that the electrode 102 can be delivered within curved or bifurcated luminal spaces.

In this embodiment, the delivery tube assembly is sequentially provided with an insulating tube 202, a channel tube 203, an outer catheter 204, and a stress tube 205 along the radial direction of the delivery tube assembly; the first end of the outer catheter 204 is fixedly connected to the electrode assembly 10, and the second end of the outer catheter 204 can be fixedly connected to the handle assembly 30; the stress tube 205 is disposed at the connection position between the outer catheter 204 and the handle assembly 30; part of the wires electrically connected to the electrode 102 can extend into the insulating tube 202, and the insulating tube 202 is configured to isolate the wires electrically connected to adjacent electrodes 102 from each other; a gap is provided between the inner wall of the channel tube 203 and the outer wall of the insulating tube 202, and one end of the channel tube 203 is connected to the electrode assembly 10 to direct fluid to the location of the electrode assembly 10 through the channel tube 203.

In this embodiment, the traction structure 201 is a mandrel, with two electrodes 102 having a mandrel passing through their interiors. An insulating tube 202 can be sleeved over an upper portion of the mandrel, and a channel tube 203 can be movably sleeved over an outer portion of the insulating tube 202. The outer side of the channel tube 203 is connected to the outer catheter 204, which is fixedly connected to the handle assembly 30 with a stress tube 205 sleeved over it. By arranging the stress tube 205, the stress at the connection between the outer catheter 204 and the handle assembly 30 can be uniformly distributed, preventing the outer catheter 204 from breaking due to stress and thereby extending its service life; the outer catheter 204 is preferably a composite reinforced tube structure such as a braided tube or a coiled tube.

In this embodiment, at least one position sensing device 40 is further comprised, with the at least one position sensing device 40 disposed inside the electrode 102 and fixedly mounted on the traction structure 201.

In this embodiment, at least one of the at least two electrodes 102 is internally insulated and equipped with a position sensing device 40. The position sensing device 40 comprises but is not limited to a positioning sensor and/or a shape-sensing optical fiber and/or a temperature sensor, used for real-time monitoring of electrode 102 position data and/or electrode shape data and/or temperature information; when the position sensing device 40 is configured as a positioning sensor, at least one positioning sensor is arranged at the first converged end 1022 of the electrode 102 disposed near the distal end of the tube body 20, for collecting position and orientation data of the electrode 102. Based on the above position coordinate information, a three-dimensional model reconstruction of the electrode 102 in space is achieved; the positioning sensor is insulated and isolated from the electrode 1021 by high dielectric strength materials such as polyimide and tetrafluoroethylene; the positioning sensor is preferably a 5DOF or 6DOF electromagnetic positioning sensor, which is capable of real-time measurement of the spatial position coordinates of the electrode assembly 10, relaying the position and orientation data to the ablation system interface for visualization.

In this embodiment, the electrode assembly 10 comprises a connecting tube 103 and two electrodes 102. The first polymeric end 1022 of the first electrode 102 is fixedly connected to a first end of a traction structure 201, and the second polymeric end 1023 of the first electrode 102 is fixedly connected to a first end of the connecting tube 103. The second end of the connecting tube 103 is fixedly connected to the first polymeric end 1022 of the second electrode 102, and the second polymeric end 1023 of the second electrode 102 is fixedly connected to a first end of a delivery tube assembly. The second end of the delivery tube assembly can be fixedly connected to the housing of a handle assembly 30, and the second end of the traction structure 201 can be fixedly connected to a wheel mechanism 301 of the handle assembly 30.

In this embodiment, the electrode assembly 10 further comprises a cap 101 and a rigid electrode 104. The first end of the cap 101 is provided with an arcuate surface, and the second end of the cap 101 is provided with a traction structure mounting position and a converged end mounting position. The traction structure mounting position is configured for fixedly connecting with one end of the traction structure 201, and the converged end mounting position is configured for fixedly connecting with the first polymeric end 1022 of the electrode 102 disposed near the distal end of a tube body 20. The first end of the rigid electrode 104 is fixedly connected to the second polymeric end 1023 of the electrode 102 disposed near the proximal end of the tube body 20, and the second end of the rigid electrode 104 is fixedly connected to the tube body 20.

In this embodiment, the cap 101 comprises an end cap 1011 and an electrode ring 1012. The arcuate surface is disposed at the first end of the end cap 1011, and the electrode ring 1012 is fixedly connected to the second end of the end cap 1011. After the electrode ring 1012 is connected to the second end of the end cap 1011, a converged end mounting position is formed between the electrode ring 1012 and the end cap 1011. The traction structure mounting position is disposed at the second end of the end cap 1011.

In this embodiment, the distal end of the end cap 1011 is an end portion 1011a, and the proximal end is a connecting post 1011b. The end cap 1011 and the electrode ring 1012 are fixedly connected by welding, clamping, bonding, or other means. The end portion 1011a of the end cap 1011 has a semicircular or conical structure, and a blind hole is provided inside the connecting post 1011b of the end cap 1011, with the traction structure 201 fixedly connected thereto. The end cap 1011 and the electrode ring 1012 are made of conductive materials, preferably metals such as medical stainless steel, nickel-titanium alloy, platinum-iridium alloy, gold, silver, etc.

In this embodiment, the area inside the connecting post 1011b where the blind hole is located constitutes the traction structure mounting position, and the annular area formed between the electrode ring 1012 and the end cap 1011 constitutes the converged end mounting position.

In this embodiment, when the electrode assembly 10 comprises two electrodes 102, the connecting wire of the electrode 102 disposed near the distal end of the tube body 20 passes through the inside of an insulating tube 202 to the interior of the housing of the handle assembly 30 for electrical connection with a positive cable, while the connecting wire of the electrode 102 disposed near the proximal end of the tube body 20 passes through the inside of the insulating tube 202 to the interior of the housing of the handle assembly 30 for electrical connection with a negative cable.

In this embodiment, the outer diameter of the electrode 102 can be extended and deformed to increase the contact area with the lesion. The conductor portion of the electrode 102 preferably comprises materials with excellent biocompatibility and corrosion resistance, such as medical stainless steel, nickel-titanium alloy, platinum-iridium alloy, gold, silver, platinum, titanium, tungsten, palladium, and other materials.

In this embodiment, the electrode 102 comprises at least one of the following features: when the electrode 102 is in a contracted state, the maximum diameter of the electrode 102 is 0.5mm-5mm; when the electrode 102 is in an expanded state, the maximum diameter of the electrode 102 is 1.5mm-20mm.

In this embodiment, when the electrode 102 is in a contracted state, the cross-section of the electrode 102 is cylindrical; when the electrode 102 is in an expanded state, the cross-section of the electrode 102 is elliptical.

In this embodiment, the outer surface of the electrode 102 is a conductive surface. During the ablation process, as the traction structure 201 brings the electrode 102 into contraction or deployment, the traction structure 201 changes the axial distance of an extended segment 1021, thereby adjusting the diameter of the extended segment 1021 to meet the wall-adhering requirements of different lesions.

In this embodiment, the electrode assembly 10 comprises at least one of the following features: the spacing between adjacent two electrodes 102 is 5mm-20mm; the length of the electrode assembly 10 is 10mm-40mm.

In this embodiment, the number of electrodes 102 comprised in the electrode assembly 10 is not limited, as long as the spacing between adjacent electrodes 102 falls within the aforementioned range and the length of the electrode assembly 10 meets the specified range.

In this embodiment, the two ends of the tubular structure are respectively the first converged end 1022 and the second converged end 1023; the first converged end 1022 and the second converged end 1023 are arranged along the axial direction of the delivery tube assembly, with the first converged end 1022 positioned near the distal end of the tube body 20 and the second converged end 1023 near the proximal end of the tube body 20; one end of the delivery tube assembly is fixedly connected to the second converged end 1023 of an electrode 102 positioned near the proximal end of the tube body 20; one end of the traction structure 201 passes through the delivery tube assembly and extends into the electrode assembly 10, fixedly connected to the first converged end 1022 of an electrode 102 positioned near the distal end of the tube body 20.

In this embodiment, the length and shape of the first converged end 1022 and the second converged end 1023 are not limited, as long as the diameters of the first converged end 1022 and the second converged end 1023 are no greater than the diameter of the expanded segment 1021 in its contracted state.

In this embodiment, the first converged end 1022 and the second converged end 1023 are made of materials that are not prone to deformation, and both are annular structures.

In this embodiment, the first converged end 1022, the expanded segment 1021, and the second converged end 1023 are either integrally formed or separately set, depending on the actual situation, and are not limited here.

In this embodiment, the electrode 102 is a tubular structure with an internal cavity composed of a grid array structure, a simply supported beam structure, a balloon structure, or the like.

In this embodiment, the electrode 102 is a tubular structure woven from metallic wires. The electrode 102 is woven from metallic wires using a weaving method of one-over-one, one-over-two, or two-over-two; the metallic wires are preferably made of metals such as medical stainless steel, nickel-titanium alloy, platinum-iridium alloy, gold, silver, and the like.

In this embodiment, the electrode 102 woven from metallic wires sequentially comprises the first converged end 1022, the expanded segment 1021, and the second converged end 1023, and the first converged end 1022, the expanded segment 1021, and the second converged end 1023 are integrally formed. Under the action of the traction structure 201, the first converged end 1022 and the second converged end 1023 can move towards each other to compress the expanded segment 1021, thereby increasing the diameter of the expanded segment 1021 and enhancing the area of contact with the lesion, or the first converged end 1022 and the second converged end 1023 can move away from each other to compress the expanded segment 1021, thereby reducing the diameter of the expanded segment 1021 and facilitating the delivery of the electrode 102.

In some other possible embodiments, the electrode 102 further comprises an expanded segment 1021, with the two ends of the expanded segment 1021 respectively fixedly connected to the first converged end 1022 and the second converged end 1023; the expanded segment 1021 comprises at least two first connecting members, which are spaced apart around the circumference of the delivery tube assembly.

In this embodiment, along the direction from the first converged end 1022 to the second converged end 1023, the surface slope of the first connecting members gradually increases from small to large and then gradually decreases again, forming an arched structure.

In this embodiment, the first connecting member is a simply supported beam. Initially, when the electrode 102 is in a contracted state, the simply supported beam closes to form a micro-arch structure. When the electrode 102 is in an expanded state, the simply supported beam bends along its thickness direction.

In this embodiment, to achieve the desired expansion form of the electrode 102, the extended segment 1021 of the simply supported beam is initially formed into an arch structure. In some embodiments, the arch structure is achieved through pre-compression assembly, where the electrode 102 is moved proximally by a traction structure 201 to maintain pre-compressed installation, causing mild bending deformation of the extended segment 1021 of the simply supported beam. When fully expanded, the traction structure 201 is moved proximally through the operating handle assembly until an ellipsoidal surface expansion is achieved. In other schemes, the arch structure can also be achieved through pre-shaping, where the simply supported beam is fixed on a shaping fixture and pre-bent shaping is achieved through high-temperature heat treatment.

In this embodiment, when the electrode 102 is in a compressed state, the length direction of the first connecting member is parallel to the axial direction of the delivery tube assembly, and the thickness direction of the first connecting member passes through the axis of the delivery tube assembly. The first connecting member comprises at least one of the following features: the thickness of the first connecting member is much smaller than its width; the width of the first connecting member is much smaller than its length; the number of first connecting member is 2-14.

In other possible embodiments, it also comprises an extended segment 1021, with both ends of the extended segment 1021 fixedly connected to a first convergence end 1022 and a second convergence end 1023, respectively. The extended segment 1021 comprises a balloon membrane and an electrode membrane sequentially arranged from the inside to the outside. The balloon membrane has a sealed cavity inside, which can communicate with the delivery tube assembly, allowing liquid to enter or exit the sealed cavity through the delivery tube assembly to drive the balloon membrane to contract or expand.

In this embodiment, the electrode membrane comprises at least two second connecting members, which are spaced apart around the circumference of the balloon membrane.

In this embodiment, the electrode membrane comprises at least one of the following features: the thickness of the electrode membrane is 10µm-100µm; the electrode membrane is a multilayer membrane structure.

In this embodiment, when the electrode 102 is in a compressed state, the length direction of the second connecting member is parallel to the axial direction of the delivery tube assembly, and the thickness direction of the second connecting member passes through the axis of the delivery tube assembly. The second connecting member comprises at least one of the following features: the thickness of the second connecting member is much smaller than its width; the width of the second connecting member is much smaller than its length; the number of second connecting members is 2-10.

Further, a traction structure 201 is penetratedly provided inside the electrode 102. An insulating tube 202 is movably sleeved on the upper part of the traction structure 201, and a channel tube 203 is movably sleeved on the outer part of the insulating tube 202. A liquid injection channel is set between the traction structure 201 and the insulating tube 202, used for infusing and expanding the balloon membrane 1021a on the electrode 102 near the distal end of the tube body 20. A liquid injection channel is set between the insulating tube 202 and the channel tube 203, used for infusing and expanding the balloon membrane 1021a on the electrode 102 near the proximal end of the tube body 20.

The various embodiments of the present application have been described above. The choice of terms used herein is intended to best explain the principles, practical applications, or technical improvements in the market of the various embodiments, or to enable other ordinary technicians in the technical field to understand the various embodiments disclosed herein.

In the absence of conflict, the features in the above embodiments and implementations can be combined with each other.

The above disclosure is merely a preferred embodiment of the present application and cannot be used to limit the scope of the present application. Therefore, equivalent changes based on the claims of the present application are still within the scope covered by the present application.

## Claims

1. A flexible electrode pulsed ablation catheter, comprising:
a tube body (20) with a distal end and a proximal end, the tube body (20) comprising a traction structure (201) and a delivery tube assembly sleeved on the traction structure;
an electrode assembly (10) located at the distal end of the tube body (20), the electrode assembly (10) comprising at least one connecting tube (103) and at least two electrodes (102), with adjacent electrodes (102) fixedly connected by the connecting tube (103), the electrodes (102) being tubular structures with internal cavities;
one end of the traction structure (201) extends through the delivery tube assembly into the electrode assembly (10) and is fixedly connected to one end of the electrode (102) near the distal end of the tube body (20);
the traction structure (201) moves axially under external force along the delivery tube assembly to bring the relative ends of the same electrode (102) towards or away from each other, causing the tubular structure to contract or expand.

2. The flexible electrode pulsed ablation catheter according to claim 1, wherein the electrode (102) comprises at least one of the following features:
the electrode (102) in a contracted state has a maximum diameter of 0.5mm-5mm;
the electrode (102) in an expanded state has a maximum diameter of 1.5mm-20mm.

3. The flexible electrode pulsed ablation catheter according to claim 2, wherein the electrode (102) in a contracted state has a cylindrical cross-section; the electrode (102) in an expanded state has an elliptical cross-section.

4. The flexible electrode pulsed ablation catheter according to claim 1, wherein the electrode assembly (10) comprises at least one of the following features:
the spacing between adjacent electrodes (102) is 5mm-20mm;
the length of the electrode assembly (10) is 10mm-40mm.

5. The flexible electrode pulsed ablation catheter according to claim 1, wherein adjacent electrodes (102) have opposite polarities.

6. The flexible electrode pulsed ablation catheter according to claim 1, wherein the tubular structure has a first aggregation end (1022) and a second aggregation end (1023);
the first aggregation end (1022) and the second aggregation end (1023) are arranged along the axial direction of the delivery tube assembly, the first aggregation end (1022) is provided near the distal end of the tube body (20), and the second aggregation end (1023) is provided near the proximal end of the tube body (20);
one end of the delivery tube assembly is fixedly connected to the second aggregation end (1023) of the electrode (102) near the proximal end of the tube body (20);
one end of the traction structure (201) extends through the delivery tube assembly into the electrode assembly (10) and is fixedly connected to the first aggregation end (1022) of the electrode (102) near the distal end of the tube body (20).

7. The flexible electrode pulsed ablation catheter according to any one of claims 1-6, wherein the electrode (102) is a tubular structure woven from metal wires.

8. The flexible electrode pulsed ablation catheter according to any one of claims 1-6, further comprising at least one position sensing device (40), the at least one position sensing device (40) is located inside the electrode (102) and fixed on the traction structure (201).

9. The flexible electrode pulsed ablation catheter according to any one of claims 1-6, wherein the hardness of the tube body (20) gradually decreases along the proximal end to the distal end of the tube body (20).

10. The flexible electrode pulsed ablation catheter according to any one of claims 1-6, wherein the tube body (20) is made of a radiopaque material.

11. The flexible electrode pulsed ablation catheter according to claim 9, wherein the delivery tube assembly sequentially comprises an insulating tube (202), a channel tube (203), an outer catheter (204), and a stress tube (205) along the radial direction of the delivery tube assembly;
the first end of the outer catheter (204) is fixedly connected to the electrode assembly (10), the second end of the outer catheter (204) is fixedly connected to a handle assembly (30), and the stress tube (205) is located at the connection position between the outer catheter (204) and the handle assembly (30);
part of the wires connected to the electrode (102) extend into the insulating tube (202), the insulating tube (202) is configured to isolate the wires connected to adjacent electrodes (102);
a gap is provided between the inner wall of the channel tube (203) and the outer wall of the insulating tube (202), and one end of the channel tube (203) is connected to the electrode assembly (10).

12. A pulsed ablation device, comprising a handle assembly (30) and the flexible electrode pulsed ablation catheter according to any one of claims 1-11;
the first end of the handle assembly (30) is electrically connected to an ablation mechanism that emits ablation energy;
one end of the delivery tube assembly is fixedly connected to the second end of the handle assembly (30), one end of the traction structure (201) extends through the delivery tube assembly and is connected to the handle assembly (30) in a driving manner, and the handle assembly (30) drives the traction structure (201) to move axially along the delivery tube assembly.

13. The pulsed ablation device according to claim 12, wherein the handle assembly (30) comprises at least two wires, a wheel mechanism (301), and a connector assembly (302);
the wheel mechanism (301) is fixedly connected to one end of the traction structure (201), and the wheel mechanism (301) drives the traction structure (201) to move axially along the delivery tube assembly under external force;
the first end of the at least two wires is electrically connected to the connector assembly (302), the connector assembly (302) is located at the first end of the handle assembly (30), and the connector assembly (302) is electrically connected to the ablation mechanism;
the second ends of the at least two wires are respectively electrically connected to the at least two electrodes (102), to enable the adjacent electrodes (102) to have opposite polarities.

14. A pulsed ablation equipment, comprising an ablation mechanism and the pulsed ablation device according to claims 12 or 13;
the ablation mechanism is configured to provide at least one pulse group (50) to the pulsed ablation device;
wherein the pulse group (50) comprises at least one high-frequency and high-voltage sub-pulse (501) and/or one low-frequency, low-voltage sub-pulse (502), the high-frequency and high-voltage sub-pulse (501) is a bipolar pulse, and the low-frequency, low-voltage sub-pulse (502) is a unipolar pulse or a bipolar pulse; wherein the bipolar pulse comprises a positive pulse amplitude and a negative pulse amplitude.

15. The pulsed ablation equipment according to claim 14, wherein the pulse group (50) comprises at least one of the following features:
the voltage amplitude range of the bipolar pulses of the high-frequency and high-voltage sub-pulses (501) is 2000V-70000V;
the pulse width of the bipolar pulses of the high-frequency and high-voltage sub-pulses (501) is 50ns-50000ns;
the number of bipolar pulses of the high-frequency and high-voltage sub-pulses (501) is 1-5000;
each voltage amplitude of the unipolar pulses and the bipolar pulses of the low-frequency, low-voltage sub-pulses (502) is 200V-3000V;
each pulse width of the unipolar pulses and the bipolar pulses of the low-frequency, low-voltage sub-pulses (502) is 10µs-500µs;
the number of each of unipolar pulses and bipolar pulses of the low-frequency, low-voltage sub-pulses (502) is 1-5000;
the number of pulse groups (50) is 1-5000.
